# EUROPEAN PATENT APPLICATION

(11) **EP 4 378 465 A1**
(43) Date of publication of application: **05.06.2024**
(21) Application number: 22849630.3
(22) Date of filing: 29.07.2022
(51) Int. Cl.: A61K 31/7064, A61K 9/08, A61K 9/19, A61K 45/00, A61P 35/00, A61P 35/02, A61P 43/00

(54) **PHARMACEUTICAL COMPOSITION FOR TREATING CANCER**

(30) Priority: 30.07.2021 JP 2021126032
(71) Applicant: SAGA UNIVERSITY, Saga-shi, Saga 840-8502 (JP); National University Corporation Kumamoto University, Kumamoto-shi, Kumamoto 860-8555 (JP)
(72) Inventor: KIMURA Shinya, Saga-shi, Saga 840-8502 (JP); KUBOTA Yasushi, Saga-shi, Saga 840-8502 (JP); MOTOYAMA Keiichi, Kumamoto-shi, Kumamoto 860-8555 (JP); HIGASHI Taishi, Kumamoto-shi, Kumamoto 860-8555 (JP); ARIMA Hidetoshi, Kumamoto-shi, Kumamoto 860-8555 (JP)
(74) Representative: J A Kemp LLP
(86) International application number: PCT/JP2022/029345
(87) International publication number: WO 2023/008570

(57) **Abstract**

Provided herein is a pharmaceutical composition for treating cancer, the pharmaceutical composition containing: a folic acid-modified hydroxyalkylated cyclodextrin selected from the group consisting of folic acid-modified hydroxypropylcyclodextrin, folic acid-modified hydroxybutylcyclodextrin, and folic acid-modified hydroxyethyl cyclodextrin; or a derivative thereof. Thus, a novel pharmaceutical composition for treating cancer and the like is provided.

## Description

### [Technical Field]

The present invention relates, inter alia, to a pharmaceutical composition for treating cancer.

### [Background Art]

Cancer is the disease that is the number one cause of death in Japan. The survival rate for some cancers is improving, but satisfactory therapies for advanced cancers are still not available and the development of more effective therapies is desired.

Cyclodextrin (CyD) is a cyclic maltooligosaccharide, in which glucose is connected by α-1,4-bonds, and is a monomolecular host molecule capable of incorporating, in a hydrophobic cavity thereof, various drugs. In addition, CyD tightly binds cholesterol and is widely used as a raft inhibitor because CyD extracts cholesterol from lipid rafts in a cell membrane and thereby disrupts a structure thereof. For example, focusing on the action against intracellular cholesterol, 2-hydroxypropyl-β-cyclodextrin (HP-β-CyD) is employed clinically for Niemann-Pick disease type C (NPC), i.e., a hereditary lipid storage disease.

In PTL 1 (WO 2014/087778) and NPL 1 (Yokoo et al., PLoS One, (2015) 10(11): e0141946.), because cholesterol is essential for the proliferation and function retention of tumor cells, hypothesis has been taken up that HP-β-CyD itself has an antitumor action, and a proliferation-inhibiting action on leukemia cells by HP-β-CyD per se has been demonstrated in vitro.

In this sphere, in addition to anticancer drugs having a high cytotoxic effect on cancer cells, a construction is anticipated in particular of drug delivery systems (DDSs) that selectively and efficiently deliver an anticancer drug to a target site. DDSs seek to optimize drug treatment by controlling the drug dynamics in the body and may be classified into control of the drug release behavior, promotion of drug absorption, targeting a drug to a target tissue, and so forth. Ligand modification with, e.g., an antibody, sugar chain, folic acid, or transferrin, is known as a method for imparting a targeting capability to a carrier.

Among these, folic acid is widely used as a ligand molecule because folic acid offers the following advantages, among others: 1) it is inexpensive; 2) the folate receptor (FR) is overexpressed in various epithelial cancer cells and exhibits low expression in normal cells, which, as a result, enables selective uptake by cancer cells via FR-mediated endocytosis; 3) repeated administration is possible due to a low antigenicity; and 4) its relatively low molecular weight thereof limits ability to influence movement of the carrier within the cell.

PTL 2 (Japanese Patent Kokai No. 2014-1204) describes the use of folic acid-appended methylated β-cyclodextrin (FA-M-β-CyD), in which folic acid is conjugated to methyl-β-cyclodextrin, as a tumor cell-selective anticancer agent.

PTL 3 (Japanese Patent Kokai No. 2016-69520) reports that a tumor-accumulative folic acid-appended cyclodextrin having an increased solubility due to the introduction thereinto of a hydrophilic substituent (for example, a polyethylene glycol group, hydroxypropyl group), is useful as a DDS with the goal of targeting cancer.

### [Citation List]

### [Patent Literature]

[PTL 1] WO 2014/087778
[PTL 2] Japanese Patent Kokai No. 2014-1204
[PTL 3] Japanese Patent Kokai No. 2016-69520

### [Non Patent Literature]

[NPL 1] Yokoo et al., PLoS One, (2015) 10(11) : e0141946.
[NPL 2] Okamatsu et al., Biomacromolecules, (2013) 14, p. 4420-4428.

### [Summary of Invention]

### [Technical Problem]

In view of the circumstances described in the preceding a novel pharmaceutical composition for treating cancer has been desired.

### [Solution to Problem]

As a result of intensive and extensive investigations in order to solve the problem identified above, the present inventors have discovered that the tropism to high folate receptor-expressing tumor cells can be enhanced, and a stronger anticancer action can be expressed, by appending folic acid to hydroxyalkylated cyclodextrin or a derivative thereof in order to endow same with a tumor cell selectivity. The present invention was achieved based on this discovery.

A pharmaceutical composition for treating cancer, inter alia, is provided here as described in the following.
[1-1] A pharmaceutical composition for treating cancer, containing a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or containing a derivative of the folic acid-appended hydroxyalkylated cyclodextrin.
[1-2] The pharmaceutical composition according to [1-1], including only a folic acid-appended hydroxyalkylated cyclodextrin or a derivative thereof as an active ingredient.
[1-3] The pharmaceutical composition according to [1-1] or [1-2], wherein the folic acid-appended hydroxyalkylated cyclodextrin or a derivative thereof is folic acid-appended hydroxypropylcyclodextrin.
[1-4] The pharmaceutical composition according to [1-3], wherein the folic acid-appended hydroxypropylcyclodextrin is folic acid-appended hydroxypropyl-β-cyclodextrin.
[1-5] The pharmaceutical composition according to any one of [1-1] to [1-4], which is used in combination with an ABL tyrosine kinase inhibitor.
[1-6] The pharmaceutical composition according to any one of [1-1] to [1-5], wherein the cancer is leukemia.
[1-7] The pharmaceutical composition according to any one of [1-1] to [1-6], which is an injectable.
[2-1] A method for treating cancer, comprising administering a composition containing a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or containing a derivative of the folic acid-appended hydroxyalkylated cyclodextrin, to a subject in need thereof.
[2-2] The method according to [2-1], wherein the composition contains only a folic acid-appended hydroxyalkylated cyclodextrin or a derivative thereof as an active ingredient.
[2-3] The method according to [2-1] or [2-2], wherein the folic acid-appended hydroxyalkylated cyclodextrin or derivative thereof is folic acid-appended hydroxypropylcyclodextrin.
[2-4] The method according to [2-3], wherein the folic acid-appended hydroxypropylcyclodextrin is folic acid-appended hydroxypropyl-β-cyclodextrin.
[2-5] The method according to any one of [2-1] to [2-4], wherein the composition is administered in combination with an ABL tyrosine kinase inhibitor.
[2-6] The method according to any one of [2-1] to [2-5], wherein the cancer is leukemia.
[2-7] The method according to any one of [2-1] to [2-6], wherein the composition is an injectable.

### [Advantageous Effects of Invention]

A novel pharmaceutical composition for treating cancer is provided herein.

### [Brief Description of Drawings]

[Fig. 1]

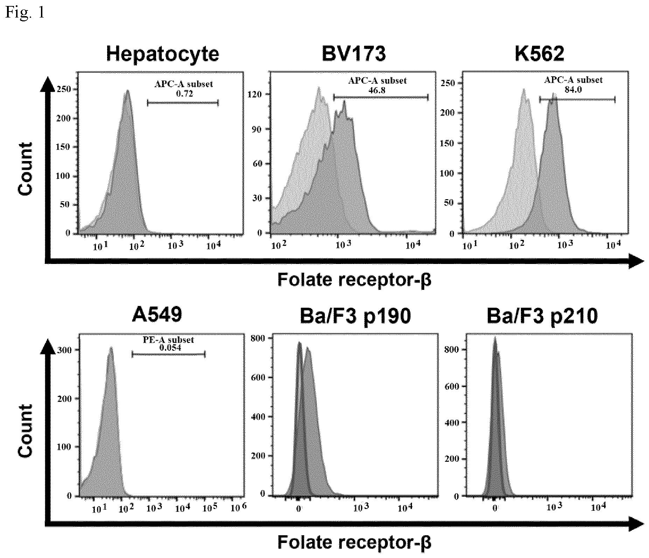

   Fig. 1 is a diagram that shows the results of the flow cytometric analysis of the expression of the folate receptor-β.
[Fig. 2]

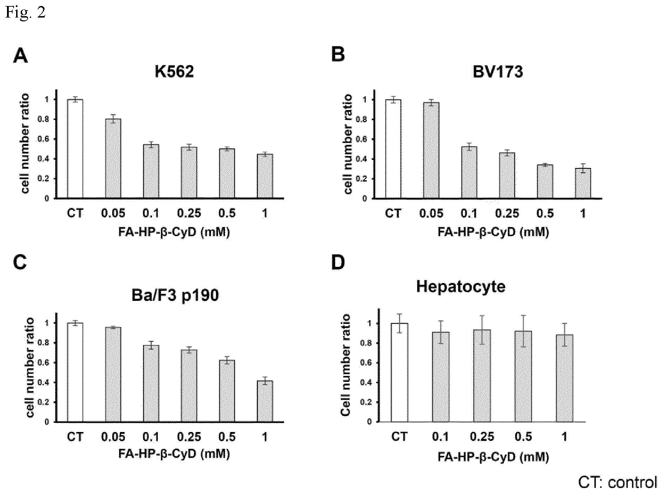

   Fig. 2 is a diagram that shows the results of an evaluation, using an MTT assay, of the proliferation inhibitory action in leukemia cell lines.
[Fig. 3]

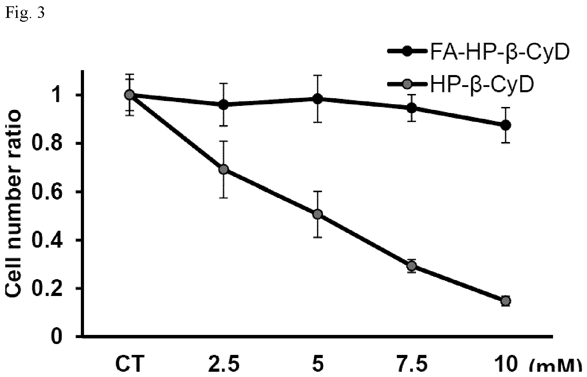

   Fig. 3 is a diagram that shows the results of an evaluation of the proliferation inhibitory action on A549 low folate receptor-expressing cells.
[Fig. 4]

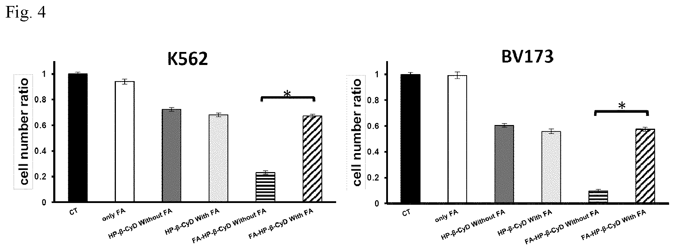

   Fig. 4 is a diagram that shows the results of an evaluation of the proliferation inhibitory action in leukemia cell lines.
[Fig. 5]

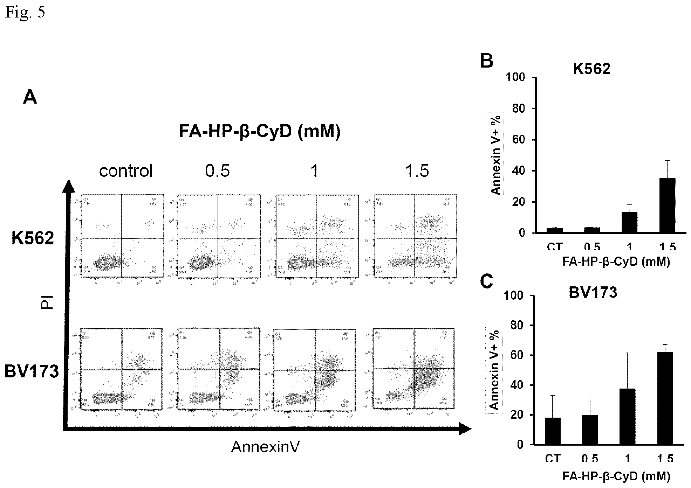

   Fig. 5 is a diagram that shows the results of an evaluation of cell apoptosis using Annexin V/PI staining.
[Fig. 6]

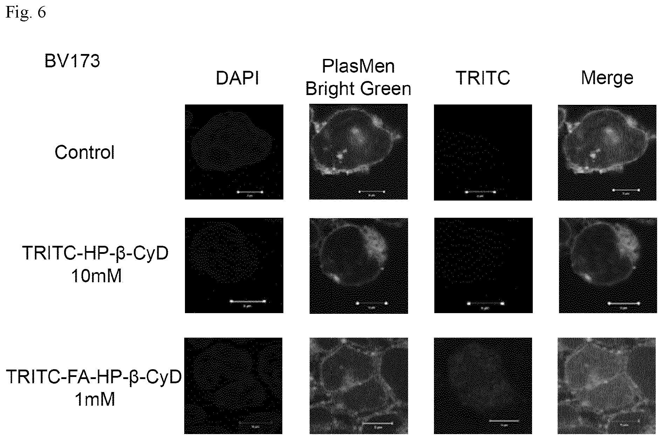

   Fig. 6 is a diagram that shows the cellular uptake of FA-HP-β-CyD.
[Fig. 7]

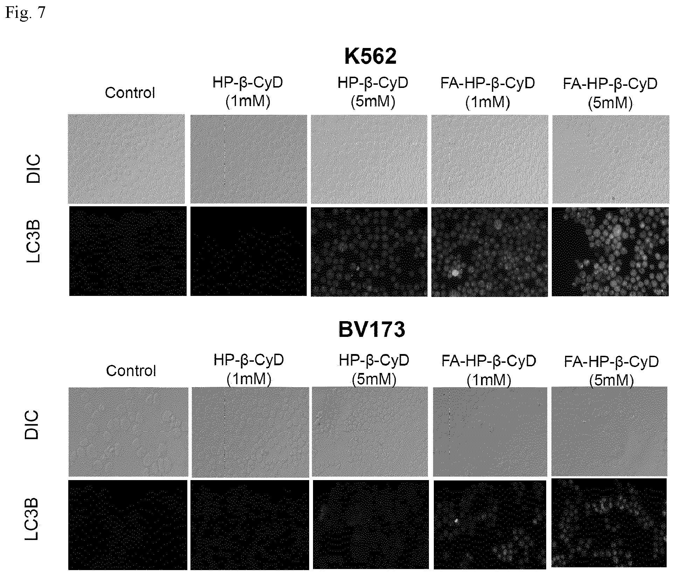

   Fig. 7 is a diagram that shows that FA-HP-β-CyD induces autophagosome formation.
[Fig. 8]

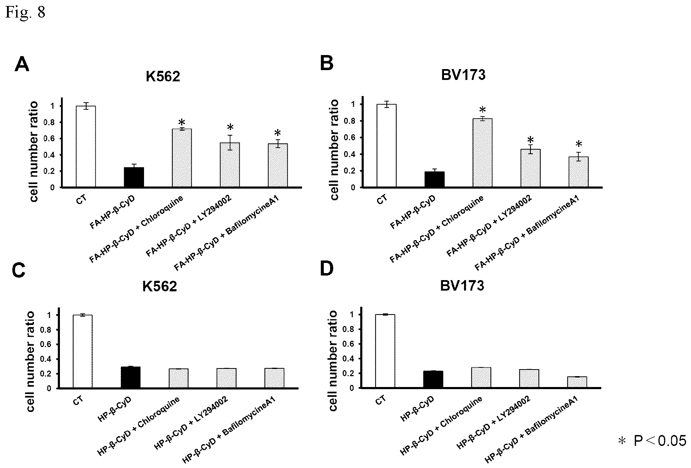

   Fig. 8 is a diagram that shows the weakening of the cell proliferation inhibitory effect of FA-HP-β-CyD due to an inhibition of autophagy.
[Fig. 9]

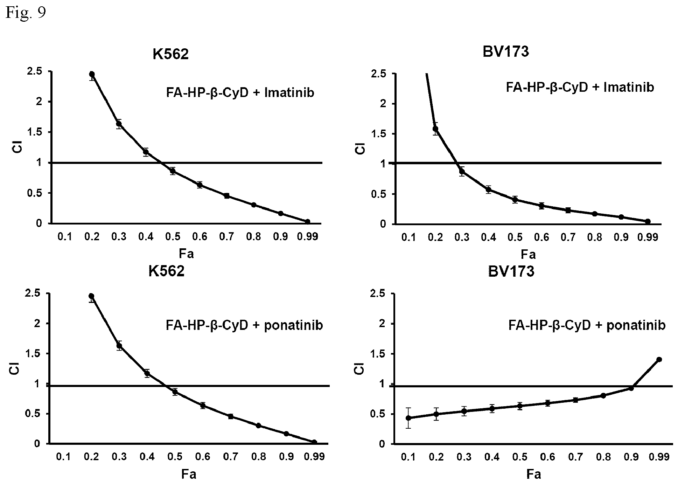

   Fig. 9 is a diagram that shows the results of calculation, using CalcuSyn software, of the effect of the combined use (combination index, CI) of FA-HP-β-CyD with imatinib or ponatinib.
[Fig. 10]

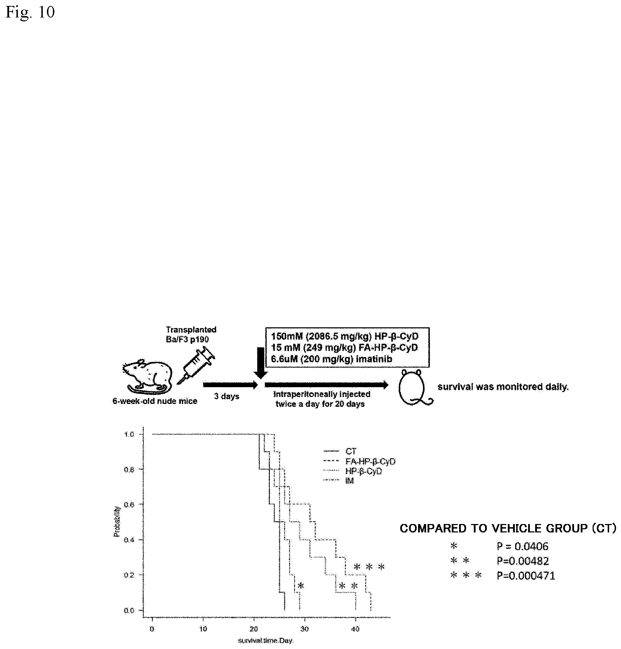

   Fig. 10 is a diagram that shows that FA-HP-β-CyD causes an elongation of the survival time in a BCR-ABL leukemia mouse model.
[Fig. 11]

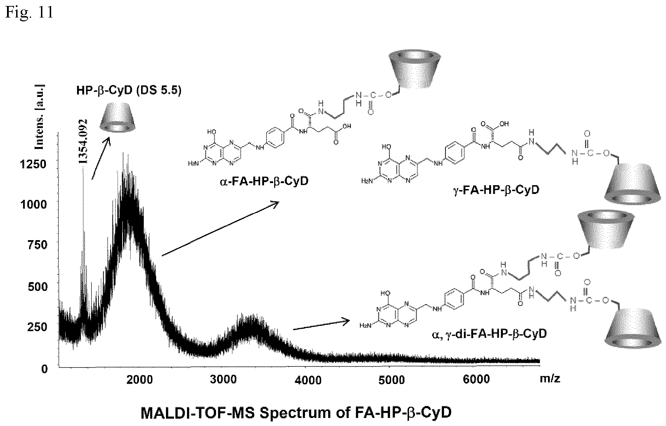

   Fig. 11 is the MALDI-TOF-MS spectrum of FA-HP-β-CyD (Example 1).
[Fig. 12]

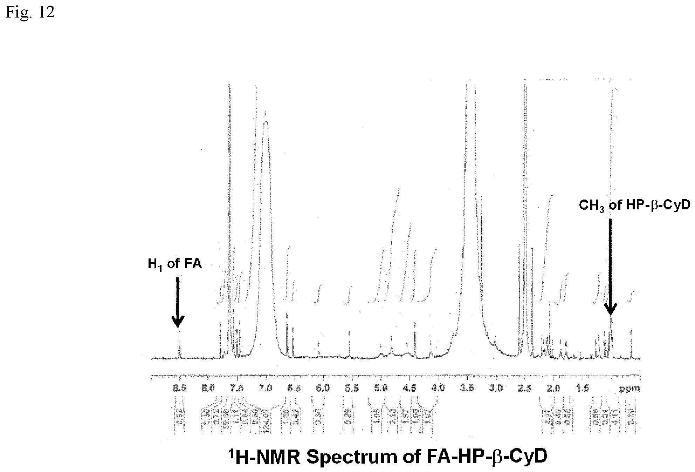

   Fig. 12 is the ¹H-NMR spectrum of FA-HP-β-CyD (Example 1).
[Fig. 13]

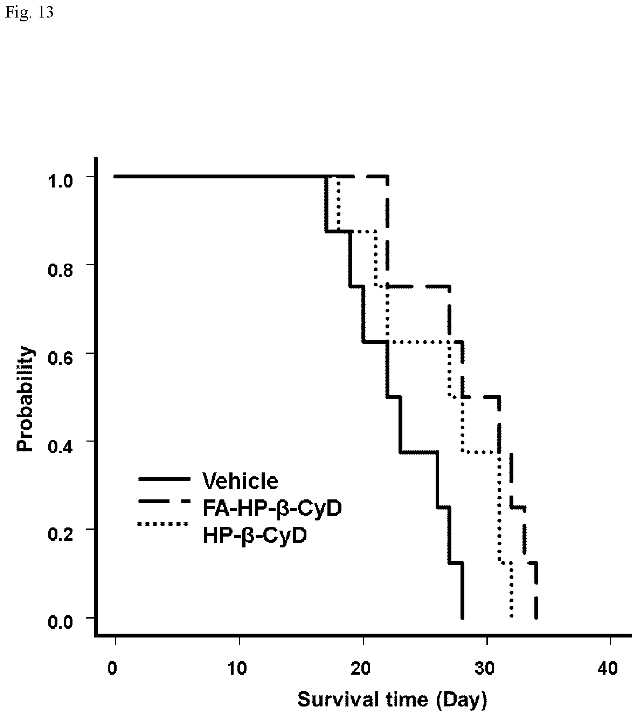

   Fig. 13 is a diagram that shows that FA-HP-β-CyD brings about an extension in the survival time in a mouse ventricular invasion model (single administration model).
[Fig. 14]

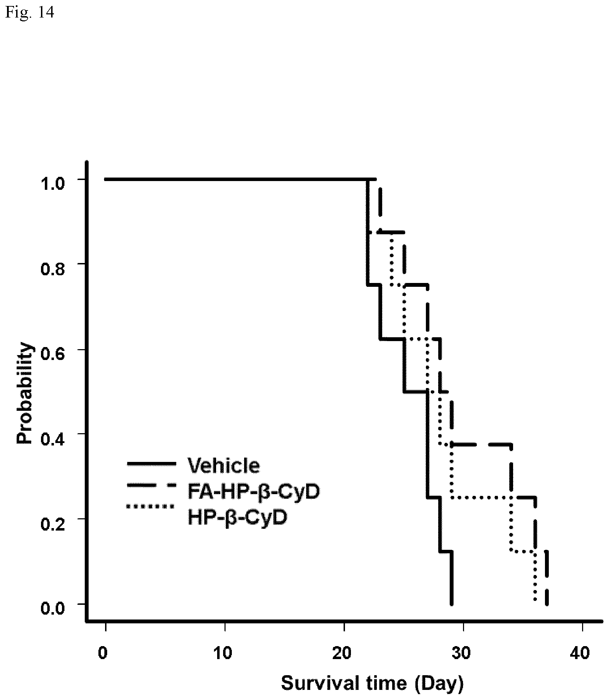

   Fig. 14 is a diagram that shows that FA-HP-β-CyD brings about an extension in the survival time in a mouse ventricular invasion model (two-time administration model).

### [Description of Embodiments]

A detailed description follows.

### Summary

The present inventors thought that the tropism to high folate receptor-expressing tumor cells could be increased, and a stronger anticancer action could be expressed, by appending folic acid to hydroxyalkylated cyclodextrin in order to endow same with a tumor cell selectivity.

The present inventors first produced folic acid-appended HP-β-CyD (FA-HP-β-CyD), which is a folic acid-appended hydroxyalkylated cyclodextrin (Example 1). In addition, it was discovered that, relative to the un-appended HP-β-CyD, this FA-HP-β-CyD exhibits the equivalent anti-leukemia action at approximately one-tenth the amount (Example 3). Moreover, it was demonstrated that there is almost no cellular intake of HP-β-CyD in the absence of appending, while, as a consequence of appending with folic acid, HP-β-CyD is incorporated in large amounts into the cell (Example 7, Fig. 6) and autophagic cell death is the main factor in the anti-leukemia action (Example 8, Fig. 7). Using a leukemia mouse model, a significant extension of the survival time due to the administration of FA-HP-β-CyD was also observed (Example 11 and Fig. 10).

It is known that leukemia stem cells, which cause the recurrence of leukemia, acquire resistance to various agents utilizing autophagy. The folic acid-appended hydroxyalkylated cyclodextrin provided herein takes advantage of this and supports the development of a novel cancer treatment.

### The folic acid-appended hydroxyalkylated cyclodextrin

"Cyclodextrin : CyD" is a cyclic maltooligosaccharide in which glucose is connected by α-1,4-bonds, and is a monomolecular host molecule capable of incorporating various drugs in its hydrophobic cavity. For example, α-CyD, which has 6 glucose residues, β-CyD, which has 7 glucose residues, γ-CyD, which has 8 glucose residues, δ-CyD, which has 9 glucose residues, ε-CyD, which has 10 glucose residues, and derivatives of the preceding are known for CyD.

The "hydroxyalkylated cyclodextrin" is a hydroxyalkylated cyclodextrin selected from the group consisting of hydroxypropylcyclodextrin, hydroxybutylcyclodextrin, and hydroxyethylcyclodextrin. A hydroxyalkylated cyclodextrin is a compound in which the hydroxyl groups of a cyclodextrin (for example, α-cyclodextrin, β-cyclodextrin, γ-cyclodextrin, and so forth) are randomly substituted by a hydroxypropyl group, hydroxybutyl group, or hydroxyethyl group. For example, hydroxyalkylated β-cyclodextrin is a compound in which hydroxyl groups at positions 2, 3, and 6 of the 7 glucoses in β-cyclodextrin have been randomly substituted with a hydroxyalkyl group.

The "folic acid-appended hydroxyalkylated cyclodextrin" is a compound in which folic acid is covalently bonded to the glucose in a hydroxyalkylated cyclodextrin. There are no particular limitations on the position at which the folic acid is bonded, but position 2 and/or position 6 on glucose are preferred and position 6 is particularly preferred. The proportion at which folic acid is bonded in the folic acid-appended hydroxyalkylated cyclodextrin is not particularly limited, but, considered as cyclodextrin molecule : folic acid molecule, 1 : 1 to 1 : 14 is preferred and 1 : 1 to 1 : 7 is more preferred. Folic acid-appended hydroxyalkylated β-cyclodextrin is a preferred folic acid-appended hydroxyalkylated cyclodextrin.

The folic acid-appended hydroxyalkylated cyclodextrin is selected from the group consisting of folic acid-appended hydroxypropylcyclodextrin, folic acid-appended hydroxybutylcyclodextrin, and folic acid-appended hydroxyethylcyclodextrin. The folic acid-appended hydroxyalkylated cyclodextrin is preferably a folic acid-appended hydroxyalkylated β-cyclodextrin selected from the group consisting of folic acid-appended hydroxypropyl-β-cyclodextrin, folic acid-appended hydroxybutyl-β-cyclodextrin, and folic acid-appended hydroxyethyl-β-cyclodextrin. Or, the folic acid-appended hydroxyalkylated cyclodextrin is preferably a folic acid-appended hydroxypropylcyclodextrin. The folic acid-appended hydroxyalkylated cyclodextrin is particularly preferably folic acid-appended hydroxypropyl-β-cyclodextrin.

### Methods for producing the folic acid-appended hydroxyalkylated cyclodextrin

The folic acid-appended hydroxyalkylated cyclodextrin can be synthesized using publicly known methods. For example, a folic acid-appended hydroxyalkylated α-cyclodextrin or folic acid-appended hydroxyalkylated β-cyclodextrin can be synthesized by aminating the hydroxyl groups of a hydroxyalkylated α-cyclodextrin or hydroxyalkylated β-cyclodextrin followed by bonding with folic acid via a condensation reaction. Or, for example, a folic acid-appended hydroxyalkylated γ-cyclodextrin can be synthesized by aminating the hydroxyl groups of a hydroxyalkylated γ-cyclodextrin followed by bonding with folic acid via a condensation reaction.

In the folic acid-appended hydroxyalkylated cyclodextrin, the hydroxyalkylated cyclodextrin may be introduced at either the α-carboxyl group or γ-carboxyl group of folic acid. For example, in the case of the introduction of hydroxyalkylated β-cyclodextrin onto folic acid, any of the following may be contained in the folic acid-appended hydroxyalkylated cyclodextrin: α-folic acid-appended hydroxyalkylated β-cyclodextrin, in which hydroxyalkylated β-cyclodextrin has been introduced at the α-carboxyl group of folic acid; or γ-folic acid-appended hydroxyalkylated β-cyclodextrin, in which hydroxyalkylated β-cyclodextrin has been introduced at the γ-carboxyl group of folic acid; or α,γ-di-folic acid-appended hydroxyalkylated β-cyclodextrin, in which hydroxyalkylated β-cyclodextrin has been introduced at both the α-carboxyl group and γ-carboxyl group of folic acid. In the case of the synthesis of a folic acid-appended hydroxyalkylated cyclodextrin in which the hydroxyalkylated cyclodextrin is introduced at the α-carboxyl group of folic acid, synthesis can be carried out by protecting the γ-carboxyl group of folic acid, followed by bonding with the folic acid and removal of the protection group. Or, in the case of the synthesis of a folic acid-appended hydroxyalkylated cyclodextrin in which the hydroxyalkylated cyclodextrin is introduced at the γ-carboxyl group of folic acid, synthesis can be carried out by protecting the α-carboxyl group of folic acid, followed by bonding with the folic acid and removal of the protection group.

In certain embodiments of the pharmaceutical composition, at least one (one, two, or all three) of α-folic acid-appended hydroxyalkylated β-cyclodextrin, γ-folic acid-appended hydroxyalkylated β-cyclodextrin, and α,γ-di-folic acid-appended hydroxyalkylated β-cyclodextrin is contained as a folic acid-appended hydroxyalkylated β-cyclodextrin.

### Derivatives of the folic acid-appended hydroxyalkylated cyclodextrin

The folic acid-appended hydroxyalkylated cyclodextrin derivatives include derivatives in which another compound is additionally covalently bonded to a folic acid-appended hydroxyalkylated cyclodextrin. The other bonded compound is not particularly limited and can be exemplified by compounds that have the ability to bind to various receptors, compounds that have an antitumor or anticancer capability, and so forth. Specific examples of such compounds are, for example, transferrin, epidermal growth factor (EGF), arginine-glycine-aspartic acid (RGD) peptide, antibodies that specifically recognize cancer cells, and so forth.

The folic acid-appended hydroxyalkylated cyclodextrin derivatives encompass derivatives in which another compound is covalently bonded to the folic acid-appended hydroxyalkylated cyclodextrin, but do not encompass inclusion derivatives with another compound.

### The pharmaceutical composition

The above-described folic acid-appended hydroxyalkylated cyclodextrin or derivative thereof can be used as a pharmaceutical composition for treating cancer by formulation in accordance with conventional methods.

A pharmaceutical composition for treating cancer, containing a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or containing a derivative of the folic acid-appended hydroxyalkylated cyclodextrin, is provided herein.

The pharmaceutical composition for treating cancer contains the folic acid-appended hydroxyalkylated cyclodextrin or derivative thereof in the composition as an anticancer active ingredient. That is, the folic acid-appended hydroxyalkylated cyclodextrin or derivative thereof is incorporated in the pharmaceutical composition as an anticancer active ingredient as such, rather than the folic acid-appended hydroxyalkylated cyclodextrin or derivative thereof being incorporated in the pharmaceutical composition as a solubilizing agent and/or stabilizer for another active ingredient. As a consequence, a pharmaceutical composition that contains folic acid-appended hydroxyalkylated cyclodextrin or a derivative thereof as a solubilizing agent and/or stabilizer for another active ingredient, is excluded from the aforementioned pharmaceutical composition for treating cancer.

As long as the pharmaceutical composition for treating cancer contains the folic acid-appended hydroxyalkylated cyclodextrin or derivative thereof as an active ingredient having an anticancer action, it may also contain another active ingredient that has an anticancer action. In certain embodiments the folic acid-appended hydroxyalkylated cyclodextrin or derivative thereof is incorporated as the main active ingredient having an anticancer action, and more preferably only the folic acid-appended hydroxyalkylated cyclodextrin or derivative thereof is incorporated as an active ingredient having an anticancer action.

The composition for treating cancer may be a composition for oral administration or a composition for parenteral administration.

For example, the composition for oral administration includes solid dosage forms and liquid dosage forms, for which specific examples are tablets (including sugar-coated tablets and film-coated tablets), pills, granules, powders, capsules (including soft capsules), syrups, emulsions, suspensions, and so forth. Such compositions are produced by methods publicly known as such and contain carriers, diluents, or excipients as commonly used in the formulation field. For example, lactose, starch, sucrose, magnesium stearate, and so forth may be used as a carrier or excipient for tablets.

For example, injectables, suppositories, eye drops, nasal drops, atomized inhalants, liniments, patches, and so forth may be used for the composition for parenteral administration, and the injectables encompass dosage forms such as intravenous injectables, subcutaneous injectables, intradermal injectables, intramuscular injectables, drip injectables, and intra-articular injectables. Such injectables are prepared according to methods publicly known as such, for example, by dissolving, suspending, or emulsifying the aforementioned active component in a sterile aqueous or oily liquid commonly used for injectables. For example, physiological saline, isotonic solutions containing glucose and/or other adjuvants, and so forth may be used for the aqueous solution for injection, and a suitable solubilizing aid, for example, alcohol (e.g., ethanol), a polyalcohol (for example, propylene glycol, polyethylene glycol), a nonionic surfactant [for example, polysorbate 80, HCO-50 (polyoxyethylene (50 mol) adduct of hydrogenated castor oil)], and so forth may be co-used. For example, sesame oil, soy oil, and so forth may be used as the oily liquid, and benzyl benzoate, benzyl alcohol, and so forth may be co-used as a solubilizing aid. The prepared injection solution is typically filled into a suitable ampoule. Suppositories for rectal administration are prepared by mixing the aforementioned active component with a conventional suppository base. The eye drops, nasal drops, inhalant sprays, suppositories, liniments, patches, and so forth are also prepared using the aforementioned active component according to methods publicly known as such.

The pharmaceutical composition for treating cancer is preferably an injectable. The injectable may take the form of either an aqueous formulation or a freeze-dried formulation, that is, a water-based injectable or a freeze-dried injectable that is dissolved at the time of use.

In certain embodiments, the pharmaceutical composition is an anticancer agent that contains the folic acid-appended hydroxyalkylated cyclodextrin or derivative thereof as the main active ingredient, and preferably as the only active ingredient, having an anticancer activity, and takes the form of an injectable. In addition to the folic acid-appended hydroxyalkylated cyclodextrin or derivative thereof, the injectable may also contain an additive as commonly used in injectables. That is, this injectable contains the folic acid-appended hydroxyalkylated cyclodextrin or derivative thereof and a pharmaceutically acceptable additive or is composed of only the folic acid-appended hydroxyalkylated cyclodextrin or derivative thereof and a pharmaceutically acceptable additive. These injectables may also take the form of either a water-soluble formulation or a freeze-dried formulation.

The cancer targeted for treatment with the pharmaceutical composition can be exemplified by breast cancer, small cell lung cancer, colon cancer, malignant lymphoma, leukemia, testicular cancer, ovarian cancer, pancreatic cancer, lung cancer, pharyngeal cancer, laryngeal cancer, tongue cancer, gum cancer, esophageal cancer, stomach cancer, bile duct cancer, kidney cancer, bladder cancer, uterine cancer, and prostate cancer. The cancer targeted for treatment is preferably leukemia.

The effective dose of the folic acid-appended hydroxyalkylated cyclodextrin or derivative thereof in the pharmaceutical composition is selected as appropriate depending on the nature of the cancer, the extent of the disease, the treatment plan, the extent of metastasis, and the size of the tumor, as well as the weight, age, sex, and racial (genetic) background of the patient, and the dose is generally determined based on factors such as the clinically observed symptoms, the degree of disease progression, and so forth. When administered to humans, the daily dose is, for example, 0.5 g/kg to 10 g/kg (30 g to 600 g for adults weighing 60 kg), 1 g/kg to 10 g/kg, 1 g/kg to 5 g/kg, or 1.2 to 5 g/kg. Administration may be carried out once or divided into multiple doses, or may be carried out continuously over time by, e.g., drip infusion. In certain embodiments, the pharmaceutical composition is administered by drip infusion over several hours to about 10 hours. Furthermore, administration may be daily or intermittently, and can be selected as appropriate depending on the condition of the subject who will receive the administration. In certain embodiments, the administration is intermittent administration.

### Co-use

In certain embodiments, the pharmaceutical composition is used in combination with another active component. In certain different embodiments, the pharmaceutical composition is used without being combined with another active component.

This "combination with another component" indicates co-use or combined use. The folic acid-appended hydroxyalkylated cyclodextrin or derivative thereof and other component may take the form of a single formulation as provided by a simultaneous formulation or may take the form of two or more formulations as provided by separate formulation. In the case of the form of two or more formulations, these may be used by simultaneous administration to the patient or may be used via separate administrations to the patient.

The additional active component can be, for example, an additional anticancer agent. This additional anticancer agent can be exemplified by the following: ABL tyrosine kinase inhibitors, EGFR inhibitors, VEGF inhibitors, mTOR inhibitors, antibody drugs, pyrimidine antagonists (e.g., cytarabine (Cylocide^{®}), enocitabine (BHAC)), purine antagonists (e.g., fludarabine (Fludara^{®}), nelarabine (Arranon G^{®}), 6-mercaptopurine (Leukerin^{®})), DNA methyltransferase inhibitors (e.g., azacitidine (Vidaza^{®})), folate antagonists (e.g., methotrexate) (Methotrexate^{®})), anthracyclines (e.g., daunorubicin (Daunomycin^{®}), idarubicin (Idamycin^{®}), doxorubicin (Adriacin^{®}), aclarubicin (Aclacinon^{®})), alkylating agents (e.g., cyclophosphamide (Endoxan^{®})), mitotic inhibitors (e.g., vincristine (Oncovin^{®}), vindesine (Fildesin^{®})), topoisomerase inhibitors (e.g., etoposide (Vepesid^{®}, Lastet^{®})), corticosteroids (e.g., prednisolone (Predonine^{®}), dexamethasone (Decadron^{®}, LenaDex^{®})), retinoic acid (Vesanoid^{®}), tamibarotene (Amnolake^{®}), anthraquinone antineoplastic agents (e.g., mitoxantrone (Novantrone^{®})), antimetabolites (e.g., hydroxyurea (Hydrea^{®})), enzyme drugs (e.g., asparaginase (Leunase^{®})), arsenic trioxide (Trisenox^{®}), interferon (Sumiferon^{®}), and immunomodulatory drugs (e.g., lenalidomide (Revlimid^{®}), pomalidomide (Pomalyst^{®})). The ABL tyrosine kinase inhibitors can be exemplified by imatinib, ponatinib, dasatinib, nilotinib, and bosutinib.

In certain embodiments, the pharmaceutical composition is used in combination with an ABL tyrosine kinase inhibitor. The ABL tyrosine kinase inhibitor in this case is preferably, e.g., imatinib, ponatinib, dasatinib, nilotinib, or bosutinib.

The effective dose of the additional active component can be selected as appropriate depending on the nature of the cancer, the extent of the disease, the treatment plan, the extent of metastasis, and the quantity of the tumor, as well as the weight, age, sex, and racial (genetic) background of the patient.

The additional active component can be made into a drug, for example, a tablet (including sugar-coated tablets and film-coated tablets), powder, granule, capsule (including soft capsules), liquid, injectable, suppository, sustained release preparation, and so forth, by mixing with a pharmacologically acceptable carrier or vehicle using a publicly known method, and these can be administered orally or parenterally.

When the pharmaceutical composition is used in combination with another active component, the blending ratio between the folic acid-appended hydroxyalkylated cyclodextrin or derivative thereof and this other component can be selected as appropriate depending on the subject for administration, the route of administration, the disease, and so forth.

### Treatment method

A cancer treatment method, comprising use of the above-described pharmaceutical composition, can also be provided here. This is preferably a cancer treatment method comprising administering a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or administering a derivative of the folic acid-appended hydroxyalkylated cyclodextrin, to a subject in need of treatment for cancer.

The specific embodiments of the pharmaceutical composition, the method of application to a specific subject, the meaning of the terminology, and so forth are as has been described above. The present treatment method may be carried in combination as appropriate with another cancer treatment method or cancer treatment drug.

The "subject" is a human or a non-human organism, for example, birds and non-human mammals (for example, cattle, monkeys, cats, mice, rats, guinea pigs, hamsters, pigs, dogs, rabbits, sheep, and horses).

The present invention also relates to the use of a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or the use of a derivative of the folic acid-appended hydroxyalkylated cyclodextrin, to produce the pharmaceutical composition that has been described in the preceding.

### Kit

A kit for treating cancer is provided here.

The kit can contain the pharmaceutical composition that has been described in the preceding. The kit can contain an instruction manual and so forth. In the case of co-use, an additional active component may be incorporated in the aforementioned pharmaceutical composition in the kit, or a separate pharmaceutical composition containing the additional active component may additionally be present in the kit.

The pharmaceutical composition and so forth are as has been described in the preceding.

All of the documents and publications mentioned in this Description are incorporated in their entirety in this Description by reference regardless of their purpose. This Description also incorporates by reference the disclosed contents of the claims, Description, and drawings of Japanese Patent Application No. 2021-126032 (filed on July 30, 2021), which is the Japanese patent application that forms the basis for the priority of the present application.

Furthermore, the objects, features, advantages, and ideas of the present invention will be apparent to persons skilled in the art from the disclosures of this Description, and the person skilled in the art will be able to readily carry out the present invention based on the disclosures in this Description. The best modes for carrying out the invention, specific examples, and so forth indicate preferred embodiments of the invention and are given for purposes of illustration or explanation, and the present invention is not limited to these. It will be apparent to the person skilled in the art that various modifications can be made based on the text of this Description within the intent and scope of the present invention disclosed by this Description.

### Examples

The present invention is described below using examples, but the present invention is not limited to or by these examples.

The various reagents used in the examples are as indicated below.

**[Table 1]**

| reagent | source |
|---|---|
| folic acid (FA) | FUJIFII,M Wako Pure Chemical Corporation |
| N,N'-dicyclohexylcarbodiimide (DCC) | FUJIFII,M Wako Pure Chemical Corporation |
| N-hydroxysuccinimide (NHS) | Tokyo Chemical Industry Co., Ltd. |
| dimethyl sulfoxide (DMSO) | FUJIFII,M Wako Pure Chemical Corporation |
| ethylenediamine (EDA) | Nacalai Tesque, Inc. |
| acetonitrile | Kanto Chemical Co., Inc. |
| HP-β-CyD | FUJIFILM Wako Pure Chemical Corporation |
| triethylamine (TEA) | FUJIFII,M Wako Pure Chemical Corporation |
| 1.1-carbonyldiimidazole (CDI) | Tokyo Chemical Industry Co., Ltd. |
| Spectra/Por 7 dialysis membrane | Repligen Corporation |

### Example 1: Preparation of folic acid-appended hydroxypropyl-β-cyclodextrin (FA-HP-β-CyD)

FA-HP-β-CyD was prepared as follows.

### (1) Synthesis of aminated folic acid (FA)

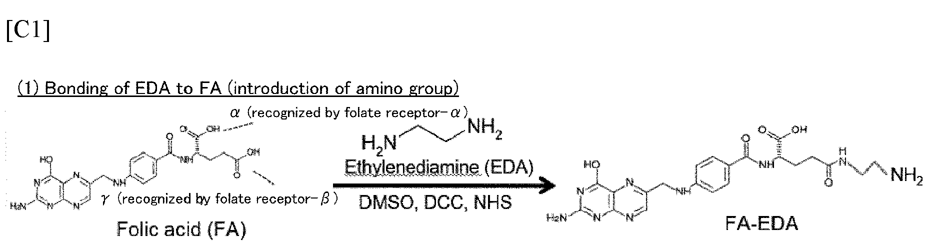

FA-EDA, which is aminated folic acid (FA), was first synthesized by bonding EDA to FA (introduction of amino group).

FA (880 mg) was dissolved in 25 mL DMSO, N,N'-dicyclohexylcarbodiimide (DCC, 412 mg) and N-hydroxysuccinimide (NHS, 230 mg) were then added, and stirring was performed for 20 hours. EDA (1 mL) was added and stirring was performed for an additional 24 hours. Washing with acetonitrile (dissolution and stirring followed by removal of supernatant) was performed 3 times, and freeze-drying was carried out to obtain FA-EDA.

### (2) Synthesis of activated HP-β-CyD

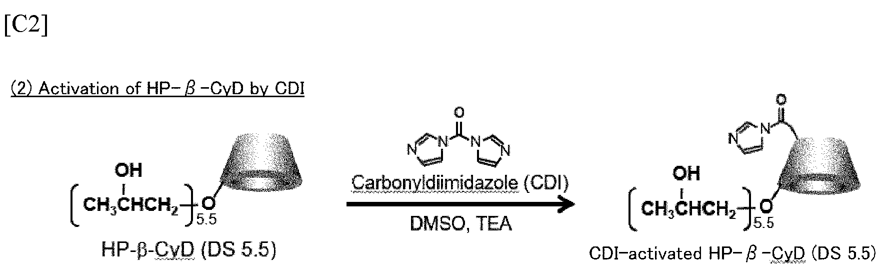

Activated HP-β-CyD was then synthesized by activating HP-β-CyD with CDI.

The HP-β-CyD (DS 5.5, 1.5 g) was dissolved in 20 mL DMSO, triethylamine (TEA, 100 µL) as a basic catalyst and CDI (1.62 g) were added, nitrogen substitution was performed, and stirring was carried out for 16 hours to obtain activated HP-β-CyD.

### (3) Reaction of FA-EDA with CDI-activated HP-β-CyD

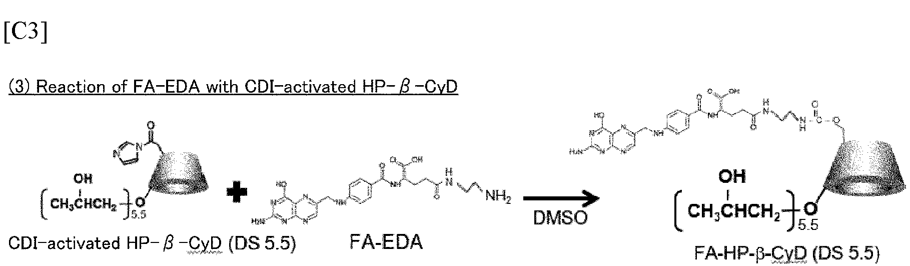

FA-HP-β-CyD was then obtained by reacting FA-EDA with the CDI-activated HP-β-CyD.

The entire amount of the FA-EDA prepared in the preceding step (2) was added to the CDI-activated HP-β-CyD solution prepared in the preceding step (1) and stirring was carried out for 24 hours. Freeze-drying was performed after dialysis (MWCO 1,000) for 1 week with 0.1 mM aqueous ammonia. After drying, FA-HP-β-CyD was obtained by fractionation on an ion-exchange resin (Diaion PK212) column.

Confirmation of the preparation was carried out using MALDI-TOF-MS and ¹H-NMR. The results of MALDI-TOF-MS are given in Fig. 11, and the results of ¹H-NMR are given in Fig. 12.

In the results for MALDI-TOF-MS in Fig. 11, two broad peaks were observed on the higher molecular weight side from HP-β-CyD (DS 5.5) by itself. The large peak in the vicinity of a molecular weight of 2,000 is thought to be FA-HP-β-CyD in which multiple FAs are bonded to one HP-β-CyD. In addition, the peak in the vicinity of a molecular weight of 3,500 is thought to be di-FA-HP-β-CyD, in which two HP-β-CyDs are bonded to one FA. The preparation of FA-HP-β-CyD could be confirmed based on these results.

The "FA-HP-β-CyD" used in the following examples contains α-FA-HP-α-CyD, γ-FA-HP-β-CyD, and α,γ-di-FA-HP-β-CyD.

The degree of substitution is given by (number of folic acid molecules/number of CyD molecules). Peaks originating with FA and HP-β-CyD were confirmed in the 1H-NMR results. The average degree of substitution by FA calculated from these peak areas was 2.08. folic acid degree of substitution = (integration value for FA / number of protons) / (number of protons for CyD/HP degree of substitution / number of protons) = (0.52/1) / (4.11/5.5/3) = 2.08

### Example 2: Expression of folate receptor-β

Expression of the folate receptor-β was analyzed by flow cytometry as follows for each of the following cell lines: a hepatocyte line, a lung cancer cell line (A549), and leukemia cell lines (BV173, K562, Ba/F3 p190, and Ba/F3 p210). PE anti-FOLR1 antibody (Bio Legend), APC anti-human Folate Receptor β (FR-β) antibody (Bio Legend), and APC anti-mouse Folate Receptor β (FR-β) antibody (Bio Legend) were added to a suspension of 2 x 10⁶ cells of the particular cell line and incubation was carried out for 20 minutes in the dark. Washing was carried out twice with 1 mL PBS (pH 7.4) and the cells were collected and filtered across a nylon mesh and analyzed using an FACSVerse flow cytometer system (BD Biosciences).

The results are given in Fig. 1. Fig. 1 shows that folate receptor-β is highly expressed by the leukemia cell lines (BV173, K562, Ba/F3 p190, and Ba/F3 p210).

### Example 3: Cell proliferation inhibitory action in leukemia cell lines

The proliferation inhibitory action in leukemia cell lines was evaluated using a modified dimethyl-thiazole-diphenyl-tetrazolium (MTT) assay as follows.

The hepatocyte line and leukemia cell lines (K562, BV173, Ba/F3 p190, Ba/F3 p210, MYL-R, K562-IMR, and KBM5-STI) were used in this example. The FA-HP-β-CyD synthesized in Example 1 was used.

The cell viability was evaluated using the trypan blue dye exclusion method. Cell proliferation was evaluated using a Cell Counting Kit-8 (Dojindo, Japan), which is an MTT assay. 2 x 10⁴ of each cell was seeded with 100 µL RPMI1640 medium (Sigma-Aldrich, USA) per well to a flatbottom 96-well plate (Greiner Labortechnik, Hamburg, Germany), and incubation for 72 hours was carried out with exposure to HP-β-CyD and FA-HP-β-CyD at different concentrations (0 mM (control : CT), 0.05 mM, 0.1 mM, 0.25 mM, 0.5 mM, and 1 mM). The data show the average ± standard deviation (SD) for 3 independent experiments.

The results of the assay using FA-HP-β-CyD are given in the following table and in Fig. 2. It is demonstrated according to Fig. 2 that cell proliferation in leukemia cell lines (K562, BV173, and Ba/F3 p190) was inhibited in a dose-dependent manner.

The results of a determination of the IC₅₀ by the assay using FA-HP-β-CyD and HP-β-CyD are given in Table 1. According to Table 1, IC₅₀ for FA-HP-β-CyD in the leukemia cell lines (K562, BV173, Ba/F3 p190, Ba/F3 p210, MYL-R, K562-IMR, and KBM5-STI) is shown to be about 1/5 to about 1/20 of that for HP-β-CyD. That is, FA-HP-β-CyD is about 5-times to 20-times stronger than HP-β-CyD. FA-HP-β-CyD is thus shown to have a stronger cytotoxic activity than HP-β-CyD.

It is predicted that the cytotoxic activity of FA-HP-β-CyD will only be about 2-times to 3-times that of HP-β-CyD when folic acid is simply appended to HP-β-CyD; however, as indicated in the preceding, the cytotoxic activity is actually about 5-times to about 20-times stronger, and this cannot be predicted based on the conventional technical knowledge.

In this regard, although the cell line is a KB cell line, which is different from that in this example, as shown in Fig. 4D of NPL 2 (Okamatsu et al., Biomacromolecules, (2013) 14, p. 4420-4428), a folic acid-appended CyD (TRITC-Fol-C₁- β-CyD) exhibited a cell uptake that was about 2- to 3-times that for the un-appended form (TRITC-NH₂-C₁-β-CyD). Since the cytotoxicity is generally assumed to increase in correlation with the intracellular concentration, it would have been heretofore expected that the cytotoxicity due to appending folic acid would be enhanced by about 2- to 3-times also for the cells used in this example.

**[Table 2]**

| Table 1 | | |
|---|---|---|
| Cell line | IC₅₀ (mM) | |
| | HP-β-CyD | FA-HP-β-CyD |
| K562 | 5.65±0.96 | 0.69±0.19 |
| BV173 | 3.46±0.33 | 0.17±0.064 |
| Ba/F3 P190 | 8.04±0.71 | 0.91±0.16 |
| Ba/F3 P210 | 9.21±0.054 | 0.96±0.017 |
| MYL-R | 8.17±0.15 | 0.93±0.11 |
| K562-IMR | 3.51±0.12 | 0.70±0.055 |
| KBM5-STI | 6.64±0.18 | 0.80±0.12 |
| hepatocyte | 12.91±2.73 | 20.10±4.50 |

### Example 4: Proliferation inhibitory action on low folate receptor-expressing cells

The proliferation inhibitory action on A549, a low folate receptor-expressing cell, was evaluated as follows.

A human non-small cell lung cancer cell line (A549) was used in this example. The FA-HP-β-CyD synthesized in Example 1 was used.

For cell proliferation, 2 x 10⁴ cells of the leukemia cell line were seeded with 100 µL DMEM medium (Sigma-Aldrich, USA) per well to a flatbottom 96-well plate (Greiner Labortechnik, Hamburg, Germany), and incubation for 72 hours was carried out with exposure to HP-β-CyD and FA-HP-β-CyD at different concentrations (0 mM (control : CT), 2.5 mM, 5 mM, 7.5 mM, and 10 mM). Evaluation was then carried out using a dimethyl-thiazole-diphenyl-tetrazolium (MTT) assay (Nacalai Tesque, Japan). The data show the average ± standard deviation (SD) for 3 independent experiments.

The results are shown in Fig. 3. While HA-HP-β-CyD exhibits a strong cytotoxic activity on A549 low folate receptor-expressing cells, FA-HP-β-CyD does not exhibit an antitumor activity. This demonstrates that FA-HP-β-CyD exhibits selectivity for cells that strongly express the folate receptor.

### Example 5: Cell proliferation inhibitory action on leukemia cell lines in the presence of folic acid (FA)

The cell proliferation inhibitory action on leukemia cell lines in the presence of folic acid (FA) was evaluated as follows using the MTT assay.

Leukemia cell lines (K562 and BV173) were used in this example. The FA-HP-β-CyD synthesized in Example 1 was used.

2 x 10⁴ of each cell with 100 µL of medium was seeded per well to a flatbottom 96-well plate (Greiner Labortechnik, Hamburg, Germany), and incubation for 2 hours at 37°C was carried out in combination with medium containing HP-β-CyD (5mM) and FA-HP-β-CyD (5 mM) in the presence or absence of FA (2 mM). Evaluation was then carried out using a Cell Counting Kit-8 (Dojindo, Japan), which is a modified dimethyl-thiazole-diphenyl-tetrazolium (MTT) assay. The data show the average ± standard deviation (SD) for 3 independent experiments.

The results are given in Fig. 4.

The evaluation here was carried out as follows, respectively, for the "CT" and "only FA" in Fig. 4. Thus, 2 x 10⁴ of each cell with 100 µL of medium was seeded per well to a flatbottom 96-well plate and incubation was carried out for 2 hours at 37°C; this was evaluated as "CT". Incubation for 2 hours at 37°C while exposed to only FA was evaluated as "only FA".

Significant difference was established as follows. A two sample t-test was performed on the agents under investigation (FA-HP-β-CyD with FA, FA-HP-β-CyD without FA). A significant difference is established when p < 0.05 in the comparison of the two samples.

As shown in Fig. 4, in leukemia cell lines (K562 and BV173), the antitumor activity of FA-HP-β-CyD in the presence of FA (FA-HP-β-CyD with FA) was significantly reduced in comparison to the system without the addition of FA (FA-HP-β-CyD without FA). This result indicates that the antitumor activity of FA-HP-β-CyD is mediated by the folate receptor.

### Example 6: Cell apoptosis

The apoptosis of cells exposed to FA-HP-β-CyD was evaluated using Annexin V/propidium iodide (PI) staining as follows.

Leukemia cell lines (K562 and BV173) were used in this example. The FA-HP-β-CyD synthesized in Example 1 was used.

The apoptosis assay was performed by staining the cells with propidium iodide (PI) and Annexin V (BD Biosciences). The cells were cultured at a cell density of 4 x 10⁵ cells/well on a 6-well plate and were incubated for 48 hours with FA-HP-β-CyD at different concentrations (0 mM (control : CT), 0.5 mM, 1 mM, and 1.5 mM). The cells were then stained with propidium iodide (PI) and Annexin V-APC (BD Biosciences) and analysis was performed using an FACSVerse flow cytometry system (BD Biosciences). The data show the average ± SD for 3 independent experiments.

The results are given in Figs. 5A, 5B, and 5C. FA-HP-β-CyD is shown to induce apoptosis in a concentration dependent manner in leukemia cell lines (K562 and BV173).

### Example 7: Cellular uptake

The cellular uptake of FA-HP-β-CyD was evaluated as follows.

A leukemia cell line (BV173) was used in this example. The FA-HP-β-CyD synthesized in Example 1 was used.

10 mg HP-β-CyD or FA-HP-β-CyD and 1 mg of TRITC were dissolved in 400 µL of dimethyl sulfoxide (DMSO) with stirring for 24 hours and the solution was then gradually poured into acetone. A precipitate was obtained and was washed with water. This was freeze-dried to prepare TRITC-HP-β-CyD and TRITC-FA-HP-β-CyD.

The cells were then cultured at a cell density of 4 x 10e5 per well on a 6-well plate and were incubated with TRITC-FA-M-β-CyD, TRITC-HP-β-CyD, FA-HP-β-CyD, or HP-βCyD at different concentrations. A Zeiss LSM880 confocal microscope was used to observe the cells. Imaris (Zeiss) fluorescent image analysis software was used for analysis.

The Control refers to incubation at the same time as the other exposure groups, but without exposure to an agent. Cell localization was stained by DAPI and the cell membrane was stained with PlasMen Bright Green, and the cellular uptake of TRITC-FA-HP-β-CyD was evaluated using confocal microscopy.

The results are given in Fig. 6. It is known that, due to their hydrophilicity and large molecular weight of approximately 1000, the cellular uptake of CyDs is generally difficult. In actuality, HP-β-CyD, which lacked appended folic acid, was not taken up by the cells according to the experiments in which TRITC was added. However, it was demonstrated by the experiments in which TRITC was added that FA-HP-β-CyD was taken up by the cells.

### Example 8: Induction of autophagosome formation

The induction of autophagosome formation by FA-HP-β-CyD was evaluated as follows.

Leukemia cell lines (K562 and BV173) were used in this example. The FA-HP-β-CyD synthesized in Example 1 was used.

The cells were cultured at a cell density of 4 x 10⁵ cells/well on a 6-well plate and were treated for 2 hours with 1 mM or 5 mM HP-β-CyD or FA-HP-β-CyD, and the cells were incubated. This was followed by use of a Cyto-ID (registered trademark) Autophagy Detection Kit (Enzo Life Sciences, USA) and the use of an Axio Imager.M2 fluorescence microscope + an ApoTome.2 (Zeiss) for cell observation.

The results are given in Fig. 7. When the cells are incubated with FA-HP-β-CyD, the autophagosome membrane protein LC3B is shown to increase in a concentration dependent manner. The DIC in Fig. 7 refers to an image with a differential interference contrast microscope. Based on the preceding, it is shown that FA-HP-β-CyD, unlike the un-appended CyDs, exhibits an antitumor activity via cellular uptake.

### Example 9: Effect of autophagy inhibition on the cell proliferation inhibitory effect

The effect of autophagy inhibition on the cell proliferation inhibitory effect was evaluated as follows.

Leukemia cell lines (K562 and BV173) were used in this example. The FA-HP-β-CyD synthesized in Example 1 was used.

2 x 10⁵ of each cell with 100 µL RPMI1640 medium (Sigma-Aldrich, USA) was seeded per well to a flatbottom 96-well plate (Greiner Labortechnik, Hamburg, Germany), and treatment was carried out for 2 hours at 37°C with 5 mM FA-HP-β-CyD and 15 mM HP-β-CyD. Pretreatment was then carried out for 24 hours with the autophagy inhibitors chloroquine (20 µM) (FUJIFILM Wako Pure Chemical Corporation), bafilomycin A1 (1 nM) (Funakoshi), or LY294002 (50 µM) (FUJIFILM Wako Pure Chemical Corporation). Cell proliferation was then evaluated using a Cell Counting Kit-8 (Dojindo, Japan), which is a modified dimethyl-thiazole-diphenyl-tetrazolium (MTT) assay. The data show the average ± standard deviation (SD) for 3 independent experiments.

The results are given in Fig. 8.

Here, the "CT" in Fig. 8 was evaluated as follows. Thus, 2 x 10⁵ of each cell with 100 µL RPMI1640 medium was seeded per well to a flatbottom 96-well plate and culture was carried out for 2 hours + 24 hours as in the exposure groups, but without exposure to the agent or autophagy inhibitor, and this was used as the CT. This CT was compared with the exposure groups.

Significant difference was determined as follows. That is, the data were analyzed using a two-sided Student's t-test without a comparison table. A value of P < 0.05 was considered to indicate a significant difference.

The cell proliferation inhibitory capability of folic acid-appended CyD (FA-HP-β-CyD) is reduced by the addition of an autophagy inhibitor (Fig. 8A and Fig. 8B), while such an effect is absent for the un-appended CyD (HP-β-CyD) (Fig. 8C and Fig. 8D). This demonstrates that, in addition to the induction of apoptosis, autophagy is strongly implicated as a mechanism for the cytotoxic activity of FA-HP-β-CyD.

### Example 10: Combined use of FA-HP-β-CyD and an ABL tyrosine kinase inhibitor

The effect of the combined use of FA-HP-β-CyD with an ABL tyrosine kinase inhibitor was evaluated as follows.

Leukemia cell lines (K562 and BV173) were used in this example. The FA-HP-β-CyD synthesized in Example 1 was used.

The IC₅₀ values of the ABL tyrosine kinase inhibitors (TKI) (imanitib and ponatinib) were obtained using the nonlinear regression program CalcuSyn (Biosoft, Cambridge, UK).

After the IC₅₀ value had been obtained for each agent, the antileukemia activity of the TKI + FA-HP-β-CyD combination was assayed using a single 96-well plate. Both K562 cells and BV173 cells were treated with five concentrations (0.25-times, 0.5-times, 0.75-times, 1.0-time, and 2.0-times the IC₅₀) of each of the compounds (imanitib and ponatinib). For comparison with the results for combined use, the cells were treated with each of the compounds (imanitib and ponatinib) as single agents. The fraction affected (Fa; an Fa of 0.25 indicates 25% viable cells) and the combination index (CI) were calculated using CalcuSyn (Biosoft). This method provides a quantification of the synergistic action (CI < 1) and the antagonist action (CI > 1) at various doses and effect levels. The data are reported as the average ± SD for 3 independent experiments.

The results are given in Fig. 9. For the K562 cells and the combination of FA-HP-β-CyD with imanitib or ponatinib, the CI value was confirmed to decline as the Fa value increased (CI < 1). For the BV173 cells and the combination of FA-HP-β-CyD with imanitib, the CI value was again confirmed to decline as the Fa value increased (CI < 1), while CI < 1 was confirmed in the region of low Fa values for the combination of FA-HP-β-CyD with ponatinib. This shows that FA-HP-β-CyD exhibits a synergistic action through co-use with drugs for treating chronic myeloid leukemia, i.e., ABL tyrosine kinase inhibitors.

### Example 11: Survival extension in a BCR-ABL leukemia mouse model

The effect due to FA-HP-β-CyD on the extension of survival in a BCR-ABL leukemia mouse model was evaluated as follows.

The FA-HP-β-CyD synthesized in Example 1 was used in this example.

EGFP + Ba/F3 BCR-ABLWT cells (1 x 10⁶ cells) were transplanted into 6-week-old nude mice. Three days after transplantation, 200 µL vehicle (n = 10), 150 mM (2086.5 mg/kg) HP-β-CyD (n = 10), 15 mM (249 mg/kg) FA-HP-β-CyD (n = 10), or 6.6 µM (200 mg/kg) imatinib (n = 10) was injected intraperitoneally twice a day for 20 days. The survival time was monitored daily. The survival data were analyzed using a log-rank nonparametric test and are reported as Kaplan-Meier survival curves.

Significant difference was determined as follows. That is, the data were analyzed using a two-sided Student's t-test without a comparison table. A value of P < 0.05 was considered to indicate a significant difference.

The results are given in Table 10. In a BCR-ABL leukemia mouse model, FA-HP-β-CyD is shown to bring about the same survival time extension at one-tenth the amount of HP-β-CyD. In vitro, FA-HP-β-CyD has a cell-killing effect that is approximately 5-times to approximately 20-times that of HP-β-CyD due to a tropism for tumor cells (Example 3). It is thought that this also results in a significant elongation of the survival time in vivo at a smaller quantity.

### Example 12: Therapeutic effect of folic acid-appended HP-β-CyD on leukemic invasion of the central nervous system

### [Objective]

Infiltration of the central nervous system by leukemia cells is one of the complications that greatly affects the prognosis for leukemia patients. Even at present, with prophylaxis for central nervous system invasion having become generalized, central nervous system invasion is still a major problem, and central nervous system invasion is reported to accompany 30 to 40% of acute lymphoblastic leukemia recurrences in children and 1 to 15% in adults. Treatment methods include intrathecal administration of anticancer drugs and intensive chemotherapy using the administration of large doses of anticancer drugs that readily transfer into the central nervous system, such as cytarabine and methotrexate. Systemic administration in particular tends to also cause damage to organs other than the central nervous system, and intrathecal administration can also cause neurotoxicity and at times cytopenia. Above all, while these treatments have been carried out for about 30 to 40 years, progress in the treatment of leukemic central nervous system invasion has been entirely lacking.

Here, the effect of HP-β-CyD and FA-HP-β-CyD on leukemic central nervous system invasion was test by constructing a mouse model.

### [Experimental method]

### Ventricular invasion mouse model

BRJ mice (female) were anesthetized (intraperitoneal administration (IP) of 100 µL of a triple anesthetic of medetomidine hydrochloride (0.75 mg/kg), mitazolam (4 mg/kg), and butorphanol tartrate (5 mg/kg)), and intrathecal transplantation was carried out, through the right coronal suture at a point 1 mm to the right of the center line defined by the sagittal suture, with a polyethylene stop tube-equipped microsyringe designed to inject BV173 cells (leukemia cells) precisely to a depth of 3 mm using a Hamilton syringe LT type needle tip style pst-3 and 25 gauge.

### Setting the dose

The FA-HP-β-CyD synthesized in Example 1 was used in this example.

The doses were set at 30 mg/kg for HP-β-CyD and 3 mg/kg for FA-HP-β-CyD by referring to the dose of HP-β-CyD for a mouse model of the hereditary lipid storage disease Niemann-Pick disease type C (NPC) (Int. J. Mol. Sci. 2021, 22, 452. https://doi.org/10.3390/ijms22010452).

In addition, based on the amount of mouse cerebrospinal fluid given in the following table, the intraventricular agent concentration at the time of agent injection becomes 7.78 mM for HP-β-CyD and 0.79 mM for FA-HP-β-CyD.

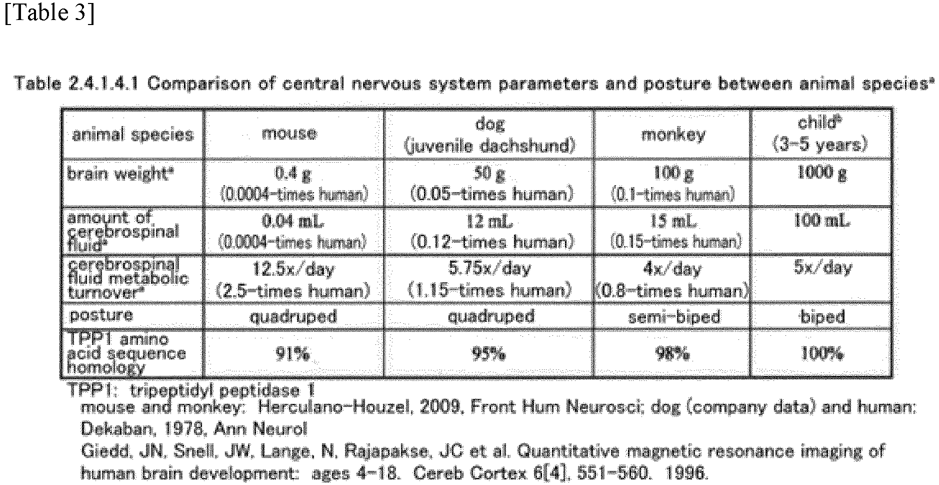

### [Results]

The results are given in Fig. 13. A single administration of vehicle (PBS administration) (n = 8), HP-β-CyD at 30 mg/kg (n = 8), or FA-HP-β-CyD at 3 mg/kg (n = 8) was made in the second week after the ventricular transplantation of the leukemia cells (BV173). An extension of survival was confirmed for the FA-HP-β-CyD group in comparison to the vehicle group and HP-β-CyD group. Here, P = 0.0746 for HP-β-CyD and P = 0.0111 for FA-HP-β-CyD, versus the vehicle group.

Additional results are given in Fig. 14. Vehicle (PBS administration) (n = 8), HP-β-CyD at 30 mg/kg (n = 8), or FA-HP-β-CyD at 3 mg/kg (n = 8) was administered a total of two times, once at the first week after ventricular transplantation of the leukemia cells (BV173) and once at the second week. An extension of survival was confirmed for the FA-HP-β-CyD group in comparison to the vehicle group and HP-β-CyD group. Here, P = 0.182 for HP-β-CyD and P = 0.0472 for FA-HP-β-CyD, versus the vehicle group. Compared with the model in which the vehicle (administration of PBS), HP-β-CyD, or FA-HP-β-CyD was administered one time in the second week after ventricular transplantation (Fig. 13), an extension in survival was not observed for the HP-β-CyD group while survival was extended by one day in the FA-HP-β-CyD group.

Significant difference was determined as follows. That is, the data were analyzed using a two-sided Student's t-test without a comparison table. A value of P < 0.05 was considered to indicate a significant difference.

### [Remarks]

As stated in the Objective, leukemic invasion of the central nervous system is a complication that greatly affects patient prognosis, and it occurs at a certain rate even with preventive measures. These treatment methods have also been unchanged for many years and progress has been absent. In addition, both intrathecal administration and systemic chemotherapy cause adverse events and cannot be frequently repeated any number of times. In this regard, because cyclodextrin has less potential to cause the cytopenia seen with the administration of anticancer drugs, and because long-term intrathecal administration of HP-β-CyD to children with Niemann-Pick disease type C is possible, it is clearly shown that folic acid-appended HP-β-CyD, which is highly effective in smaller doses, is a promising drug against leukemic invasion of the central nervous system.

## Claims

1. A pharmaceutical composition for treating cancer, comprising a folic acid-appended hydroxyalkylated cyclodextrin selected from the group consisting of folic acid-appended hydroxylpropylcyclodextrin, folic acid-appended hydroxylbutylcyclodextrin, and folic acid-appended hydroxylethylcyclodextrin, or comprising a derivative of the folic acid-appended hydroxyalkylated cyclodextrin.

2. The pharmaceutical composition according to claim 1, comprising only a folic acid-appended hydroxyalkylated cyclodextrin or a derivative thereof as an active ingredient.

3. The pharmaceutical composition according to claim 1 or 2, wherein the folic acid-appended hydroxyalkylated cyclodextrin or a derivative thereof is folic acid-appended hydroxypropylcyclodextrin.

4. The pharmaceutical composition according to claim 3, wherein the folic acid-appended hydroxypropylcyclodextrin is folic acid-appended hydroxypropyl-β-cyclodextrin.

5. The pharmaceutical composition according to any one of claims 1 to 4, which is used in combination with an ABL tyrosine kinase inhibitor.

6. The pharmaceutical composition according to any one of claims 1 to 5, wherein the cancer is leukemia.

7. The pharmaceutical composition according to any one of claims 1 to 6, which is an injectable.
